# EUROPÄISCHE PATENTSCHRIFT

(11) **EP 2 373 991 B1**
(45) Veröffentlichungstag und Bekanntmachung des Hinweises auf die Patenterteilung: **15.10.2014**
(21) Anmeldenummer: 10710994.4
(22) Anmeldetag: 05.01.2010
(51) Int. Cl.: G01N 33/00, G01N 27/66

(54) **MESSGERÄT UND VERFAHREN ZUM ERFASSEN DES GEHALTES VON ÖL, KOHLENWASSERSTOFFEN UND OXIDIERBAREN GASEN IN LUFT ODER DRUCKLUFT**
MEASURING MACHINE AND METHOD FOR DETECTING THE CONTENT OF OIL, HYDROCARBONS, AND OXIDIZABLE GASES IN AIR OR COMPRESSED AIR
APPAREIL DE MESURE ET PROCÉDÉ POUR DÉTECTER LA TENEUR EN HUILE, HYDROCARBURES ET GAZ OXYDABLES DANS L'AIR OU L'AIR COMPRIMÉ

(30) Priorität: 05.01.2009 DE 102009004278
(43) Veröffentlichungstag der Anmeldung: 12.10.2011
(73) Patentinhaber: Synthesechemie Dr. Penth GmbH, 66822 Lebach (DE)
(72) Erfinder: PENTH, Bernd, 66822 Lebach (DE); PENTH, Max, 66822 Lebach (DE); PENTH, Felix, 66822 Lebach (DE)
(74) Vertreter: Vièl, Christof
(86) Internationale Anmeldenummer: PCT/DE2010/075000
(87) Internationale Veröffentlichungsnummer: WO 2010/075858

(56) Entgegenhaltungen:
- DE-A1- 2 407 940
- RU-C1- 2 340 889
- US-A- 3 762 878
- US-A- 3 933 432
- US-A1- 2007 051 163

## Beschreibung

Die Erfindung betrifft ein Messgerät und ein Verfahren zum Erfassen des Gehaltes von Öl, Kohlenwasserstoffen und oxidierbaren Gasen in Luft oder Druckluft.

Es gibt verschiedene Sensortechniken zur Erfassung von Kohlenwasserstoffen in Luft oder Druckluft. Häufig verwendet werden elektrisch beheizte Halbleiteroxidmaterialien. Diese Halbleiteroxide verändern im beheizten Zustand ihren elektrischen Widerstand in Abhängigkeit von der Menge der in der Luft enthaltenen Kohlenwasserstoffe.

Eine andere Methode ist die Erfassung der Kohlenwasserstoffe mittels Pellistoren. Dazu wird der zu messende Gasstrom über eine kleine Kugel aus beheiztem Katalysatormaterial geleitet, in deren Innerem sich eine beheizte Platinwendel befindet. Die Messung der Kohlenwasserstoffmenge lässt sich erfassen durch die Änderung des elektrischen Widerstandes der beheizten oder einer zweiten Platinwendel, die sich durch die Verbrennungswärme des Kohlenwasserstoffes am Katalysator einstellt.

Ebenfalls verwendet werden Flammenionisationsdetektoren. Hierbei werden Kohlenwasserstoffe in einem Gasstrom verbrannt und die Spannungsänderung zwischen zwei Elektroden in der Flamme gemessen.

Eine weitere Methode ist die Erfassung der Kohlenwasserstoffe mittels Photoionisation. Dabei werden die Kohlenwasserstoffe mit starkem ultraviolettem Licht bestrahlt. Die Energiemenge des Lichtes muss dabei so hoch sein, dass Elektronen aus dem Kohlenwasserstoff herausgeschlagen werden. Deren Menge lässt sich über zwei Elektroden messen. Die mindestens erforderliche Photonenenergie beträgt für aromatische Kohlenwasserstoffe 8,5 bis 9,2 eV, für brennbare Kohlenwasserstoffe mindestens 9,0 bis 12,6 eV.

Die mittels Photoionisationsdetektoren generierten Messwerte lassen meist nur indirekt auf die gemessene Stoffmenge schließen, da die Messwerte auch von der Formel der Verbindung abhängen und selbst bei gleichen Summenformeln recht stark variieren können. Sofern die zu messende Verbindung aber konstant, bekannt und möglichst auch einheitlich ist, lässt sich die Konzentration des Kohlenwasserstoffes recht gut messen.

Allerdings sinkt die Messgenauigkeit mit abnehmender Konzentration an Kohlenwasserstoffen. Insbesondere steigt dabei der Einfluss des Feuchtegehaltes der Luft. Mit abnehmendem Kohlenwasserstoffwert wird der Einfluss der Luftfeuchte somit zunehmend größer. Messungen von Kohlenwasserstoffmengen im unteren mg / m³ - Bereich und insbesondere im µg / m³ - Bereich sind demnach nicht ausreichend genau durchzuführen.

Problematisch für genaue Messungen sind weiterhin die Drift der Nulllinie des Sensors und die Drift der Empfindlichkeit in Abhängigkeit von der Zeit, sowie teilweise auch deren Temperaturabhängigkeit.

Für die unterschiedlichen Anwendungen von Druckluft werden unterschiedliche Grenzwerte für den Ölanteil gefordert. Ölanteile bestehen aus tröpfchenförmigen Ölaerosolen und aus Öldämpfen. Ölaerosole und Öldämpfe können durch verschiedene Verfahren aus dem Druckluftstrom eliminiert werden.

Die Messung von Öl in Druckluft ist aber eine bislang nicht befriedigend gelöste Aufgabe.

Es gibt Druckluftströme, die einen hohen Ölgehalt von weit über 10 mg / m³ bis in den Bereich von einigen g / m³ Luft besitzen, bei denen der Ölgehalt vorwiegend aus Ölaerosolen besteht. Wegen des Tröpfchencharakters von Aerosolen lassen sich diese Ölgehalte mit der Messtechnik, die in diesem Konzentrationsbereich für Kohlenwasserstoffdämpfe verwendet wird, wie beispielsweise Halbleitersensoren nur sehr unzuverlässig oder überhaupt nicht messen. Die Ölaerosole lagern sich nämlich unoxidiert oder teiloxidiert als teerartiges Katalysatorgift auf dem Sensor ab.

Andere Druckluftströme sind bereits durch Filter oder Katalysatoren so weit aufgearbeitet, dass die Aerosole weitgehend entfernt sind, so dass im Luftstrom nur noch gasförmige Ölanteile vorhanden sind.

Öle haben einen niedrigen Dampfdruck, so dass für die reinen Öldämpfe Konzentrationen von unter 10 mg / m³ Luft typisch sind. Messgeräte zur Erfassung von Kohlenwasserstoffgehalten, die in der Regel auf Halbleitersensoren oder auf Infrarotsensoren basieren, messen aber erst oberhalb von 10 mg / m³ Luft mit akzeptabler Genauigkeit und Reproduzierbarkeit.

Stand der Technik zur diskontinuierlichen Messung von ölhaltiger Luft sind DIN/ISO 8573-2 und DIN/ISO 8573-5. Aerosole und Dämpfe werden demnach in Probennahmesystemen auf Glasfasern und Aktivkohle abgeschieden und diese zur Ermittlung des Ölgehaltes an ein zertifiziertes Labor gesendet.

In der DE 691 22 357 T2 und der dort zitierten US 4 891 186 A wird die Analyse auch von Kohlenwasserstoffen mittels Flammenionisationsdetektoren von Messgasen und Bezugsgasen als Referenz und deren Auswertung beschrieben.

Die DE 33 12 525 A1 beschreibt eine Vorrichtung zur Messung des Aufteilungsverhältnisses verzweigter Gasströme.

In der DE 41 20 246 A1 wird das von einem Flammenionisationsdetektor zu messende Gas mittels einer Vermischungsapparatur verdünnt, um die Explosionsgefahr zu senken.

Die DE 24 07 940 A beschreibt eine Vorrichtung zur gleichzeitigen Messung des Anteils der Kohlenwasserstoff-Fraktion mit einem niedrigen Siedepunkt und der gesamten Kohlenwasserstoffe ohne diese Fraktion in einer Gasprobe.

Die US 2007/0051163 A1 betrifft eine Vorrichtung zum Übermitteln von verschiedenen Gasproben an einen Gassensor in einer Detektionszone ohne substantielle Änderung des Druckes der Probe.

Das Zumischen von bestimmten Reagenzgasen in einer kombinierten Photoionisations-Ionenmobilitätsspektrometrie zur Detektion von schwach protonenaffinen Substanzen ist aus der DE 196 09 582 C2 bekannt.

DE 197 12 823 A1 beschreibt einen Infrarot-Gasanalysator mit einem integrierten, nicht näher beschriebenen Oxidationskatalysator. Wegen des großen Störeinflusses von Wasser wird auch eine Feuchtigkeitsbeseitigungseinrichtung verwendet, die die zu messende Luft für referenzierende Messungen trocknen kann.

In der DE 197 12 823 A1 und dem dort zitierten vorigen Stand der Technik werden ein Katalysator und Magnetventile als Bauteile wie in der vorliegenden erfindungsgemäßen Anmeldung verwendet. Zielsetzung, Messmethode, Vorgehensweise, logische Verschaltungen und Ergebnisse sind jedoch völlig verschieden.

Zur zuverlässigen und akzeptierten Messung sind Vergleichsmessungen mit Prüfgasen aus Gasflaschen Stand der Technik. Prüfgase sind aber nur bedingt geeignet, insbesondere wenn die zu messenden Kohlenwasserstoffgehalte sehr niedrig sind. Ursache dafür ist der zunehmende Einfluss der oft sogar schwankenden Luftfeuchtigkeiten auf die Signale der zu messenden sehr niedrigen Kohlenwasserstoffgehalte in der zu messenden Druckluft, während die Prüfgase prinzipiell trocken sind, bzw. einen konstanten Feuchteghalt haben.

Der Einfluss der Feuchtigkeit kann einerseits die Nulllinie verschieben, zusätzlich aber kann die Messempfindlichkeit sich verändern.

In einigen Industriebereichen wird nach DIN ISO 8573-1 aber beispielsweise der Nachweis verlangt für die Einhaltung eines Grenzwertes von 0,01 mg / m³ Luft für Druckluft der Klasse 1. Nach DIN ISO 8573-1 ist für Druckluft der Klasse 0 sogar ein Restölgehalt von < 0,01 mg / m³ Luft bestimmt.

Die bekannten Messprinzipien und die dazugehörigen Sensoren konnten bislang für diese Anwendung nicht eingesetzt werden.

Es wurde zwar bereits versucht, den Feuchtegehalt des zu messenden Luftstromes mit einem Feuchtesensor zu erfassen und mit dem Messwert für den Kohlenwasserstoffgehalt so zu verrechnen, dass der Feuchtegehalt keinen Einfluss auf die resultierende Messgröße hat. In der Praxis gestaltet sich dies aber als schwierig, da die Feuchtemessungen die hohen Genauigkeitsanforderungen nicht erfüllen. Zusätzlich ist es ein Problem, dass die wechselnden Temperaturen einen Einfluss haben.

Eine Schwierigkeit ergibt sich dadurch, dass die Einstufung eines Kohlenwasserstoffgehaltes nach DIN ISO 8573-1 in mg / m³ erfolgt. Der von einem PID gemessene Wert wird dagegen in ppm angegeben, da das Funktionsprinzip des PID-Sensors eine Abhängigkeit der Signalstärke vom Anteilsverhältnis der Kohlenwasserstoffteile zur Anzahl Luftteile bedingt. Es muss also bekannt sein, welchen Kohlenwasserstoff man messen will. Außerdem besitzen unterschiedliche Kohlenwasserstoffe unterschiedliche Responsefaktoren. Kalibriert man also einen Sensor mit beispielsweise Isobuten und misst anschließend einen anderen Kohlenwasserstoff mit dem so kalibrierten Sensor, muss der gemessene Wert entsprechend dem anderen Responsefaktor und dem anderen Molekulargewicht des anderen Kohlenwasserstoffes umgerechnet werden. Öle bestehen aber aus unterschiedlichen Anteilen einer ganzen Reihe unterschiedlicher Kohlenwasserstoffe.

Eine derzeit übliche Vorgehensweise ist die Messung von Kohlenwasserstoffen nach DIN ISO 8573-5 mit adsorbierenden Sammelröhrchen. Diese Röhrchen werden nach der Adsorptionszeit in einem zertifizierten Labor extrahiert und der Inhalt vermessen. Messungen mit diesen Sammelröhrchen führen daher erst mit großer zeitlicher Verzögerung zu Ergebnissen. Öldurchbrüche werden daher häufig erst erkannt, wenn ein Schaden durch kontaminierte Leitungen und Produkte längst eingetreten ist.

Trotz vielfältiger und jahrelanger Bemühungen der Industrie, ein geeignetes Messgerät zum Messen von Öldampf in Druckluft mit Konzentrationen unter 1 ppb zu schaffen, ist bislang ein entsprechendes Gerät nicht am Markt verfügbar. Ein Beispiel für diese erfolglosen Bemühungen mit einem Überblick findet sich in der Dissertation von N. Papamichail; "Residual Oil Monitoring in pressmised Air with SnO₂ - based Gas Sensors".

Diese Bemühungen sind auch daran gescheitert, dass das Messgerät gleichzeitig den harten Betriebsbedingungen in einer drucklufterzeugenden Umgebung standhalten soll, auch von ungeschultem Personal intuitiv und einfach zu bedienen sein soll, andererseits aber alle Anwendungsmöglichkeiten in der Bedienung zur Verfügung stellen soll, die für das Erreichen der geforderten Messgenauigkeit bedient werden müssen.

Auf dem Markt erhältlich ist auch eine Vielzahl von transportablen Messgeräten auf der Basis von Photoionisationsdetektoren, beispielsweise für Überwachungsaüfgaben in der chemischen Industrie oder bei der Feuwerwehr. Die Genauigkeit dieser Geräte bewegt sich im ppm-Bereich.

Aufgabe der vorliegenden Erfindung ist es somit, ein Messgerät zum Erfassen des Gehaltes von Öl, Kohlenwasserstoffen und oxidierbaren Gasen in Luft oder Druckluft zu entwickeln, das geeignet ist, Konzentrationen im unteren µg / m3 - Bereich, bzw. im ppb - Bereich zu detektieren und das eine Messgenauigkeit von 10 Mikrogramm Öl / m³ Luft mit einem Messfehler im einstelligen Prozentbereich ermöglicht.

Diese Aufgabe wird eifindungsgemäß dadurch gelöst, dass das Messgerät einen Luft- bzw. Druckluftanschluß und einen sich daran anschließenden Durchflussbegrenzer sowie einen sich daran anschließenden beheizbaren Oxidationskatalysator aufweist, an den sich ein Photoionisationsdetektor anschließt, wobei schaltbare Mittel zum Leiten der Luft bzw. Druckluft über den Oxidationskatalysator und an diesem vorbei direkt zum Photoionisationsdetektor vorgesehen sind und dass zwischen dem Luft- bzw. Druckluftanschluß (M) und dem Durchflussbegrenzer (D) ein schaltbarer Prüfgaseinlaß (C) angeordnet ist.

Alternativ wird die Aufgabe dadurch gelöst, dass das Messgerät einen Luft- bzw. einen Druckluftanschluss und einen sich daran anschließenden Durchflussbegrenzer und einen sich daran anschließenden Photokatalysator aufweist, an den sich ein Photoionisationsdetektor anschließt, wobei schaltbare Mittel zum Leiten von Luft, bzw. Druckluft über den Photokatalysator und an diesem vorbei direkt zum Photoionisationsdetektor vorgesehen sind und dass zwischen dem Luft- bzw. Druckluftanschluß (M) und dem Durchflussbegrenzer (D) ein schaltbarer Prüfgaseinlaß (C) angeordnet ist. Der Photokatalysator wird mit ultraviolettem Licht, vorzugsweise mit einer Wellenlänge von 370 -385 nm, angestrahlt.

Alternativ kann die Aufgabe auch dadurch gelöst werden, dass das Messgerät einen Luft- bzw. Druckluftanschluß und einen sich daran anschließenden Durchflussbegrenzer und einen sich daran anschließenden Aktivkohleadsorber aufweist, an den sich ein Photoionisationsdetektor anschließt, wobei schaltbare Mittel zum Leiten der Luft bzw. Druckluft über den Aktivkohleadsorber und an diesem vorbei direkt zum Photoionisationsdetektor vorgesehen sind.. und dass zwischen dem Luft- bzw. Druckluftanschluß (M) und dem Durchflussbegrenzer (D) ein schaltbarer Prüfgaseinlaß (C) angeordnet ist.

Die Alternative, die einen beheizten Oxidationskatalysator vorsieht, wurde als die Variante mit den genauesten Ergebnissen erkannt. Allerdings ist der Energiebedarf vergleichsweise hoch und für ein transportables Gerät weniger gut geeignet. Die Alternative, die einen Aktivkohleadsorber vorsieht, ist einfach aufgebaut, aber vergleichsweise ungenau und eher wartungsintensiv. Die Alternative, die einen Photokatalysator aufweist, ist weitgehend wartungsarm und verbraucht wegen der Verwendung von UV-LEDs weniger Energie als der thermische Oxidationskatalysator und ist daher gut für transportable Geräte geeignet.

Es liegt im Rahmen der Erfindung, dass eine Messkammer vorgesehen ist, die einen in die Kammermitte ragenden Detektoreingang für einen Photoionisationsdetektor aufweist, bei der eine Kammmerwand aus mit UV-LEDs bestücktem transparentem Kunststoff besteht, der von Kammer, Detektor und der transparenten Kunststoffwand gebildete Hohlraum kleiner als 1 ml ist und eine Öffnung mit Fließwiderstand für den periodischen Gasaustausch in dem Hohlraum vorgesehen ist.

Eine Weiterbildung der Erfindung besteht darin, dass parallel zu dem Oxidationskatalysator, dem Photokatalysator bzw. dem Aktivkohleadsorber mindestens ein Permeator zum Freisetzen einer über die Zeit konstanten, definierten Kohlewasserstoffmenge angeordnet ist, wobei schaltbare Mittel zum Leiten der Luft bzw. Druckluft über den Permeator und an diesem vorbei direkt zum Photoionisationsdetektor vorgesehen sind.

Ebenso ist es zweckmäßig, dass zwischen dem Luft- bzw. Druckluftanschluß und dem Durchflussbegrenzer ein schaltbarer Nulllufteinlaß angeordnet ist.

Eine Ausbildung der Erfindung besteht darin, dass im Anschluss an den Photoionisationsdetektor ein schaltbarer Anschluß zu einem Aktivkohleröhren vorgesehen ist.

Eine bevorzugte Ausbildung der Erfindung besteht darin, dass dem Photoionisationsdetektor ein Kompensator für die Luftfeuchtigkeit vorgeschaltet ist.

Im Rahmen der Erfindung liegt auch ein Verfahren zum Erfassen des Gehaltes von Öl, Kohlenwasserstoffen und oxidierbaren Gasen in Luft oder Druckluft unter Verwendung eines erfindungsgemäßen Messgerätes, wobei zur Durchführung einer referenzierenden Messung während der Messzeit von kohlenwasserstoffhaltigen Luft- oder Druckluftströmen ein variabler Anteil der Kohlenwasserstoffe katalytisch oxidiert wird und auf diese Weise Verdünnungsreihen erzeugt werden.

Ebenso liegt ein Verfahren zum Erfassen des Gehaltes von Öl, Kohlenwasserstoffen und oxidierbaren Gasen in Luft oder Druckluft unter Verwendung eines erfindungsgemäßen Messgerätes, wobei zur Durchführung einer referenzierenden Messung während der Messzeit der kohlenwasserstoffhaltigen Luft ein variabler Anteil Nullluft zur kohlenwasserstoffhaltigen Luft zugemischt wird und auf diese Weise Verdünnungsreihen erzeugt werden, im Rahmen der Erfindung.

Bei diesen Verfahren ist es vorteilhaft, dass der eventuell leicht sinusförmige Verlauf der Sensorantwort in der Verdünnungsphase durch Mittelwertbildung geglättet wird.

Schließlich ist es zur Erfindung gehörig, dass zum Erhöhen der Meßgenauigkeit durch den Permeator die zu messende kohlenwasserstoffhaltige Luft, als auch die katalytisch behandelte Luft, bzw. Nullluft, in gleicher Menge mit Kohlenwasserstoff angereichert wird, um den Messbereich in den linearen Messbereich zu verschieben.

Die in der vorliegenden Erfindung gefundenen Lösungen sind in den bekannten Beschreibungen und Geräten nicht realisiert. Insbesondere sind beispielsweise nicht erwähnt die Möglichkeit, einen Photokatalysator zu verwenden, beliebige Verdünnungsreihen zu generieren, die Verwendung von Permeationsröhrchen als Referenz und zur Erhöhung der Messempfindlichkeit, die Verwendung eines Feuchtepuffers (Kompensators) und Mittel, um den optimalen Ventilumschaltzeitpunkt zu erkennen, sowie die Kombinationen dieser neuen Möglichkeiten.

PID-Sensoren gelten allgemein als linear in ihrem Ansprechverhalten gegenüber schwankenden Kohlenwasserstoffkonzentrationen. Es hat sich bei den Messungen im Rahmen der Erfindung allerdings gezeigt, dass bei genaueren Messungen im Bereich von 10 Mikrogramm Öl / m³ Luft bereits eine erhebliche und mit weiter fallender Konzentration eine weit überproportional stark zunehmende Abschwächung des Messignales stattfindet.

Es war daher eine Forderung an das Messgerät, eine korrigierende Linearitätsfunktion zu entwickeln und anzuwenden.

Es zeigt sich allerdings, dass die korrigierende Linearitätsfunktion das bei sehr niedrigen Konzentrationen auftretende Genauigkeitsproblem nur teilweise löst. Da die Ursache der Abweichung von der Linearität in der Signalabschwächung liegt, führt eine Signalverstärkung zwar zu einem verbesserten Wert, allerdings ist dabei mit Fehlern durch Rauschen zu rechnen.

Es wird daher durch die Erfindung ermöglicht, auch diese durch ein überproportional schwaches Signal im Bereich von 1-10 Mikrogramm Öl / m³ Luft verursachte Ungenauigkeit zu eliminieren.

Weiterhin wurde eine portable Variante des Messgerätes geschaffen, um im mobilen Einsatz Kohlenwasserstoffmessungen in verbesserter Genauigkeit durchführen zu können.

Nachfolgend wird die Erfindung anhand von Zeichnungen näher erläutert.

Es zeigen
- Fig. 1: den schematischen Aufbau des erfindungsgemäßen Messgerätes,
- Fig. 2: die Schaltstellung der Magnetventile für die Messung von Messluft,
- Fig. 3: die Luftführung von dem Druckluftanschluß durch den Katalysator,
- Fig. 4: die Luftführung von dem Druckluftanschluß durch den Permeator,
- Fig. 5: die Luftführung von dem Druckluftanschluß durch den Katalysator und anschließend den Permeator,
- Fig. 6: die Luftführung von dem Druckluftanschluß durch den Permeator und anschließend den Katalysator,
- Fig. 7: den Messkurvenverlauf bei einer beispielhaften Messung mit Verdünnungsreihen,
- Fig. 8: eine Messkammer eines erfindungsgemäßen Messgerätes,
- Fig. 9: den Messkurvenverlauf einer Messung von aktivkohlebehandelter Druckluft
- Fig. 10: den Messkurvenverlauf einer Messung von leicht angefeuchteter Nullluft
- Fig. 11: den Messkurvenverlauf einer Messung von leicht angefeuchteter Nullluft nach dem Kompensator,
- Fig. 12: den Messkurvenverlauf einer Messung von aktivkohlebehandelter Druckluft nach dem Kompensator und
- Fig. 13: den Messkurvenverlauf einer Messung von aktivkohlebehandelter Druckluft nach dem Kompensator mit drei eingeschobenen Messungen Prüfgas.

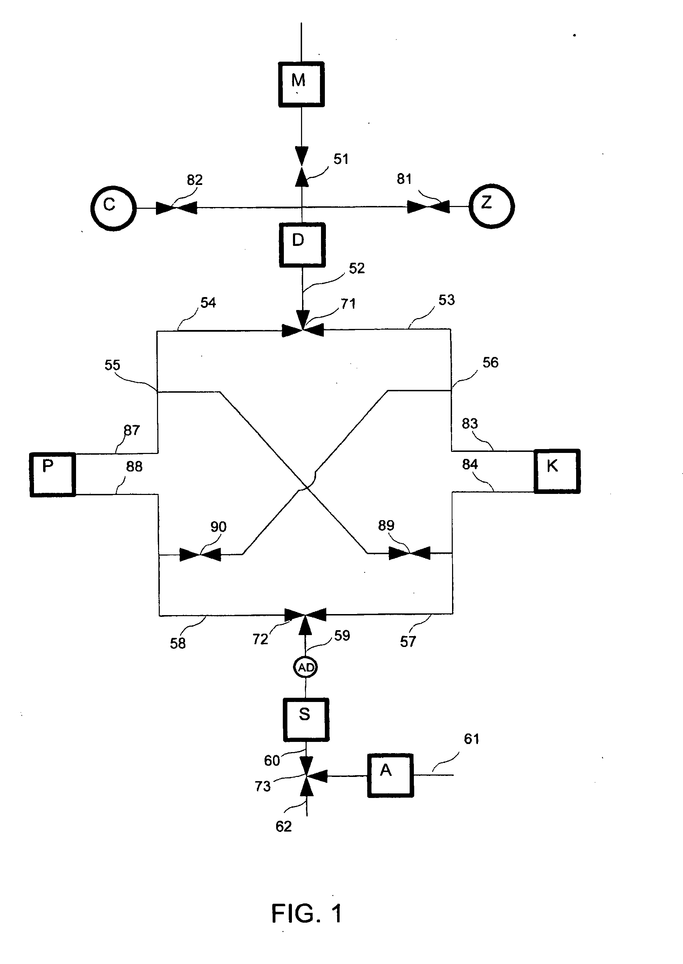

Der Aufbau des Messgerätes ist in FIG. 1 dargestellt. Die Ventile 51, 81, 82, 89, 90 sind elektrisch betätigte magnetische Absperrventile. Die Ventile 71, 72, und 73 sind elektrisch betätigte magnetische 3/2-Wegeventile.

M ist ein Druckluftanschluss mit fest eingestelltem Druckregler zwischen 1 und 14 bar, beispielsweise 7 bar.

C ist Prüfgas in einer Prüfgasflasche mit ebenfalls fest eingestelltem Druckregler, durch den beispielsweise eine Reduktion auf 7 bar erfolgt.

Z ist Nullluft in einer Nullluftgasflasche, ebenfalls mit beispielsweise auf 7 bar reduziertem Druck. In der Prüfgasflasche befindet sich Nullluft und zusätzlich ein Kohlenwasserstoff mit einer Konzentration im ppb- oder im ppm-Bereich.

D ist ein Durchflussbegrenzer. Dies kann ein "Flow restrictor" aus Sintermetall oder auch einfach eine Düse aus Saphir oder Rubin, bevorzugt mit einem Innendurchmesser zwischen 50 und 200 µm, beispielsweise mit 80 µm Innendurchmesser sein. Somit wird die Durchflussmenge an Luft auf beispielsweise 2 Liter / Minute bei 7 bar Vordruck begrenzt.

P ist ein "Permeator", in dem sich eine Permeationsmembran befindet. Dabei handelt es sich beispielsweise um ein kurzes, verschlossenes Stück Kunststoffrohr aus PTFE, gefüllt mit einem Kohlenwasserstoff. Unter der Voraussetzung einer konstanten Temperatur und eines konstanten Luftdurchlasses wird durch Permeation in der vorbei strömenden Luft ein definierter, konstanter Kohlenwasserstoffgehalt erzeugt.

K ist ein Oxidationskatalysator. Dabei handelt es sich um einen beheizten Behälter mit einer Füllung an Kupfermanganatgranulat. Zu seiner Herstellung wird ein Edelstahlrohr mit in der Katalysatorenindustrie handelsüblichem Kupfermanganatgranulat in einer Menge zwischen 2 g bis 50 g, beispielsweise Carulite 300 der Fa. Carus gefüllt. Die Oxidationstemperatur soll bei maximal 200°C liegen, damit die erforderliche Beheizung des Katalysators und der zu oxidierenden Luft gerätetechnisch und energetisch akzeptabel bleibt.

Hier muss die Tatsache berücksichtigt werden, dass die in der Industrie verwendeten Schmieröle und Kompressorenöle schwefelhaltige Komponenten enthalten, welche die üblichen, als Oxidationskatalysatoren in der KFZ-Abgasbehandlung eingesetzten Edelmetalle katalytisch vergiften.

Während im KFZ-Abgasstrang die beim Betrieb auftretenden Temperaturen von über 600°C wieder zu einer Entgiftung führen, reicht die hier gesetzte Temperaturobergrenze von 200°C zur Entgiftung, also dem Wiederablösen der Schwefelkomponente von der Katalysatoroberfläche, nicht aus.

Prinzipiell konnte für die gestellte Aufgabe auch mit Edelmetallkatalysatoren bereits bei 200°C ausreichende katalytische Aktivität festgestellt werden; allerdings ist die Lebensdauer des Katalysators wegen der Vergiftung eingeschränkt. Die Lebensdauer kann aber deutlich verlängert werden, wenn vor den Edelmetallkatalysator ein Schwefelfänger eingebaut wird. Geeignet sind beispielsweise Granulate der Oxide von Zink, Eisen, Kupfer oder Mangan. Es wurde aber auch Metallwolle aus Kupfer, Nickel und Edelstahl erfolgreich eingesetzt, sowie das Sorbens C-28 der Fa. United Catalysts. In der mobilen Variante handelt es sich beim Katalysator bevorzugt um einen Photokatalysator mit einem hohen Anteil an Titandioxid, der durch UV-LEDs (Wellenlänge 370-385 nm; Fa. Lumitronix) angestrahlt wird. Die Herstellung des Photokatalysators ist unter anderem beschrieben in DE 198 11 708 A1.

Auf der Suche nach einem geeigneten Nicht-Edelmetallkatalysator hat sich in Versuchen gezeigt, dass käufliches Kupfermanganat (Hopcalit) in Granulatform sehr gut geeignet ist und bereits bei Temperaturen ab 160°C eingesetzt werden kann. Zwar reagiert auch Kupfermanganat mit den Schwefelverbindungen in den Ölen zu katalytisch unwirksamen Verbindungen, allerdings ist die Aufhahmekapazität ungleich höher und für die gestellte Aufgabe ausreichend.

Dabei zeigte sich aber, dass Kupfermanganat als Metalloxid mit Luftfeuchtigkeit und Kohlendioxid temperaturabhängig und reversibel chemische Verbindungen eingeht. Luftfeuchtigkeit hat insbesondere im Bereich von 10 Mikrogramm Öl / m³ Luft aber einen Einfluss auf den Messwert. Für die Anforderungen an den Aufbau des Messgerätes bedeutete dies, dass die Aufnahme und die Abgabe von Feuchtigkeit aus dem Katalysatormaterial in den behandelten Luftstrom konzeptionell berücksichtigt werden muss.

**AD** ist ein Kompensator für Luftfeuchtigkeit. Zu dessen Herstellung werden in einen kleinen Behälter oder in ein Rohrstück feuchtigkeitsaufnehmende Materialien, wie Molekularsieb oder Kugeln aus Aluminiumoxid eingefüllt, wie sie typischerweise in Adsorptionstrocknern verwendet werden. Aufgabe dieses Kompensators ist es aber nicht, die durchströmenden Luftströme zu trocknen. Vielmehr funktioniert der Kompensator erst in seinem unter den jeweiligen Bedingungen (Temperatur, Feuchtigkeit, Druck) gesättigten Zustand nach Einstellen eines Gleichgewichtes zwischen Aufnahme und Abgabe optimal, gibt also Feuchtigkeit an einen trockeneren Luftstrom ab oder nimmt Feuchtigkeit aus einem feuchteren Luftstrom auf. Entsprechend der nachfolgenden Funktionsbeschreibung des Messgerätes wechseln durch das Umschalten von Magnetventilen die Luftströme in Intervallen, so dass deren leicht unterschiedlicher Feuchtigkeitsgehalt ausgeglichen wird.

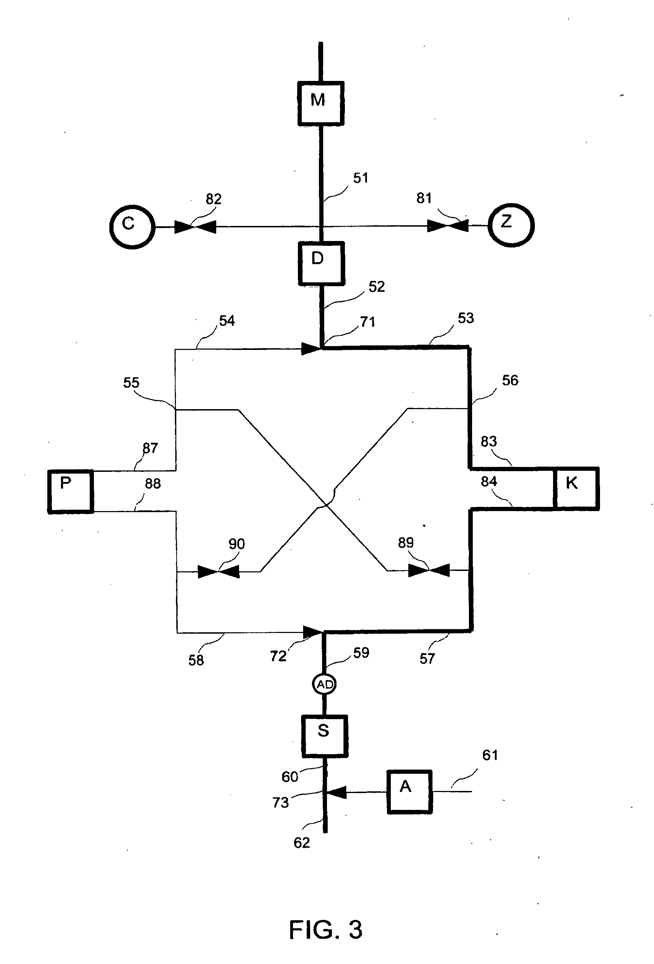

Dieser Kompensator erhöht die Messgenauigkeit des Messgerätes insbesondere im unteren Messbereich entscheidend, wenn als Katalysator ein beheizter Oxidationskatalysator, bestehend aus Kupfermanganoxid (Hopcalit) verwendet wird. Eine vereinfachte, beispielhafte Ausführung des erfindungsgemäßen Messgerätes misst, wie in **FIG. 1** und **FIG. 3** dargestellt, **M** gegen **MK** (der Permeator ist weggelassen). Da der Katalysator **K** während der Messluftphase **M** weiterbeheizt wird und dabei die Kerntemperatur der Katalysatorfüllung wegen der unterbrochenen Luftdurchströmung etwas ansteigt, spaltet das Katalysatormaterial kleine Mengen Feuchtigkeit ab. Nach dem Umschalten auf die Stellung **MK** wird der Katalysator wieder durchströmt und gibt die gesammelte Feuchtigkeit während dieser Phase zunächst wieder ab. Sobald die Phase **MK** zeitlich zu Ende geht, kühlt die Kerntemperatur des Katalysators geringfügig ab und nimmt dadurch wieder kleine Wassemiengen auf.

S ist ein Photoionisationsdetektor (PID) mit einer Photonenenergie von 10,6 eV. Besonders empfindlich ansprechende geeignete PIDs stellen die Firmen Baseline-mocon oder auch Alphasense her.

A ist ein Aktivkohleröhrchen, mit dem Proben nach DIN ISO 8573-5 genommen werden können. Da das zu diesem Röhrchen führende Magnetventil von der Recheneinheit gesteuert wird, kann die durchgeflossene Luftmenge gezielt bemessen werden und in einer optionalen Einstellung des Messgerätes für die Kalibrierung des Messgerätes der vom Messgerät ermittelte Messwert mit dem nach der DIN ISO - Methode gefundenen Messwert abgeglichen werden. Die Anwendung dieser Schaltoption, die Luftströme durch das Aktivkohleröhrchen leitet, ist für die Funktion des Messgerätes selbst aber nicht wesentlich.

Die oben beschriebene Schwierigkeit, aus der Signalstärke des PID-Sensors auf den Gehalt des Öles nach DIN ISO 8573-1 in mg / m³ zu schließen, erfolgt in dem erfindungsgemäßen Messgerät dadurch, dass in einer geräteinternen Datenbank die zu messenden Öle in ihrer prozentualen Zusammensetzung mit den dazugehörigen Molekulargewichten und Responsefaktoren hinterlegt sind, intern verrechnet und umgerechnet angezeigt werden. Der Anwender kann so vor dem Messen den Namen des zu messenden Öles eintragen und erhält als Anzeige den Messwert in mg des jeweiligen Öles / m³ Luft. Zusätzlich erhält der Anwender eine Einordnung des Messergebnisses in die Druckluftklasse und eine einstellbare Aktivierungsschwelle für den Alarmwert.
**FIG. 1** gibt keinen Hinweis auf die Schaltstellung der Ventile.

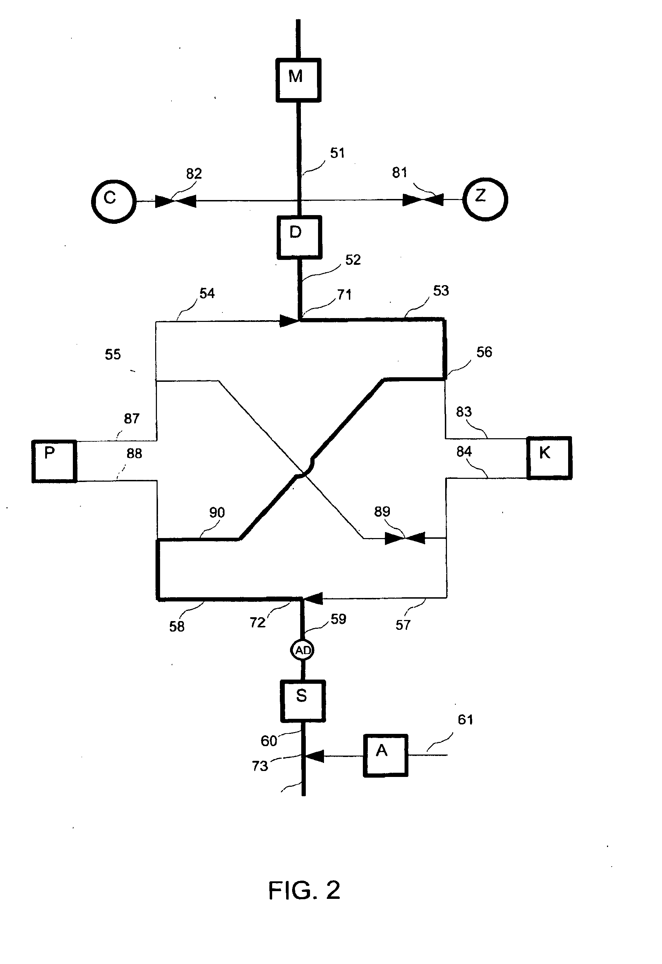

**FIG. 2** zeigt die Schaltstellung der Magnetventile für die Messung von Messluft. Die gegenüber **FIG. 1** dick gezeichneten Linien zeigen den Weg der Luft durch das Messgerät. Die hier gezeigte Schaltstellung führt die Messluft **M** direkt zum Sensor. Die verschlossene Seite der Ventile ist jeweils als Dreieck dargestellt. Die am Sensor ankommende Luft soll hier als **M** bezeichnet werden. Öffnet man statt des Ventiles 51 entweder das Ventil 82 oder das Ventil 81, wird bei ansonsten gleicher Luftführung am Sensor das Prüfgas **C** oder die Nullluft **Z** gemessen. Die dann am Sensor ankommende Luft wird der Einfachheit halber nachstehend als **C** oder **Z** bezeichnet. Analoge Bezeichnungen sollen hier auch für die anderen Luftströme gelten.
**FIG. 3** zeigt die Luftführung von **M** durch den Katalysator **K**, in dem die Kohlenwasserstoffe oxidiert werden. Die am Sensor ankommende Luft soll hier als **MK** bezeichnet werden. Öffnet man statt des Ventiles 51 entweder Ventil 82 oder Ventil 81, wird bei ansonsten gleicher Luftführung am Sensor **CK** oder **ZK** gemessen.

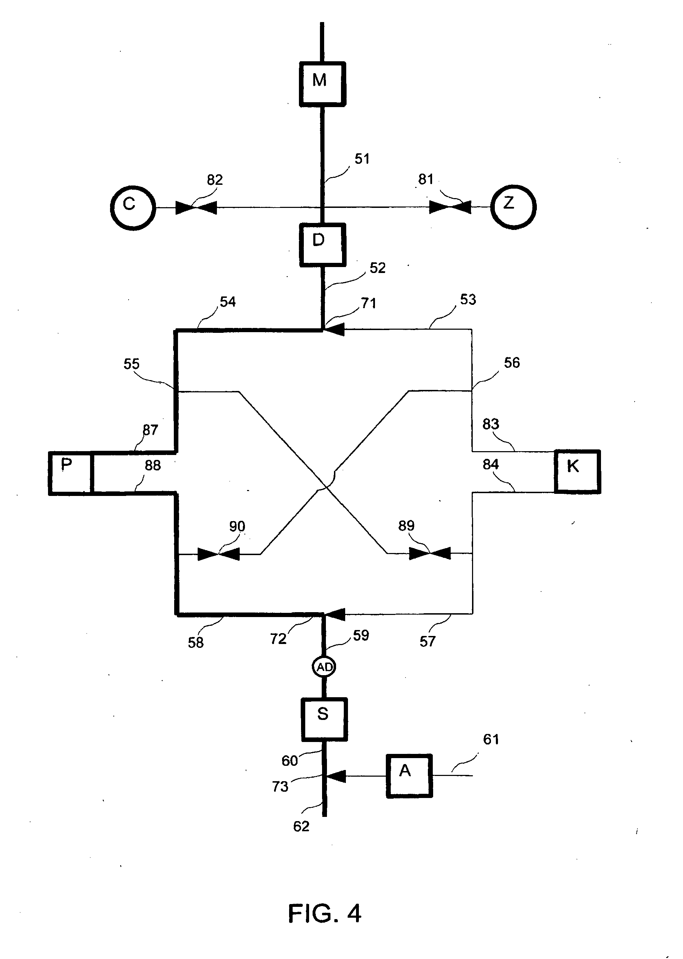

**FIG. 4** zeigt die Luftführung von **M** durch den "Permeator" **P** in dem der Luftstrom mit Kohlenwasserstoff aus dem Permeationsröhrchen angereichert wird. Die am Sensor ankommende Luft wird hier als **MP** bezeichnet. Öffnet man statt des Ventiles 51 entweder Ventil 82 oder Ventil 81, wird bei ansonsten gleicher Luftführung am Sensor das Prüfgas **C** oder die Nullluft **Z**, jedoch zusätzlich angereichert mit Kohlenwasserstoff aus dem Permeationsröhrchen gemessen. Die dann am Sensor ankommende Luft wird als **CP** oder **ZP** bezeichnet.

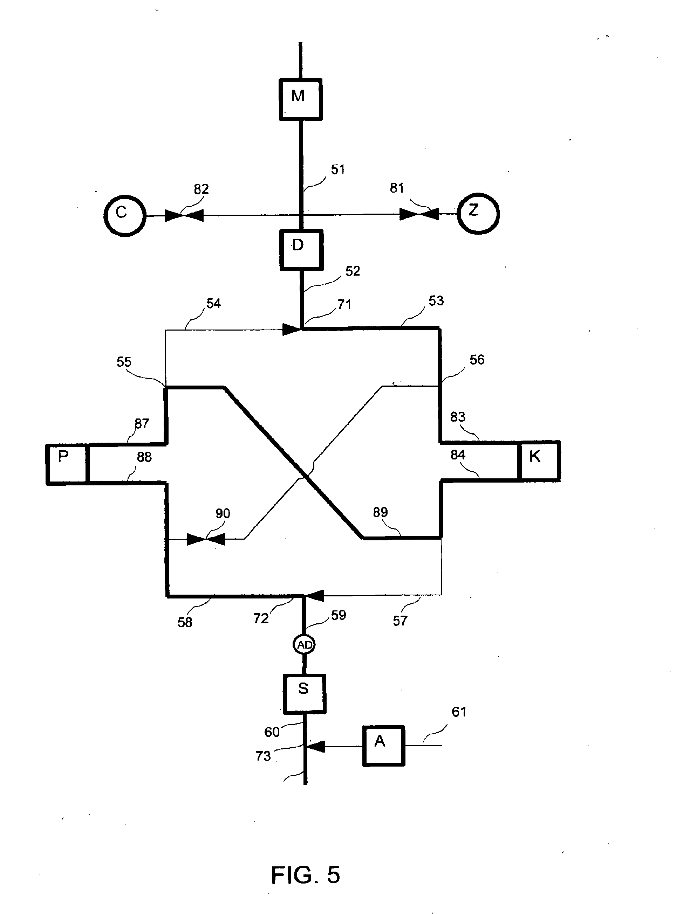

**FIG. 5** zeigt die Luftführung von **M** durch den Katalysator **K** und anschließend den Permeator **P.** Die am Sensor ankommende Luft wird dementsprechend als **MKP** bezeichnet. Öffnet man statt des Ventiles 51 entweder das Ventil 82 oder das Ventil 81, erhält man bei ansonsten gleicher Luftführung am Sensor Luft, die als **CKP** oder **ZKP** bezeichnet werden soll.

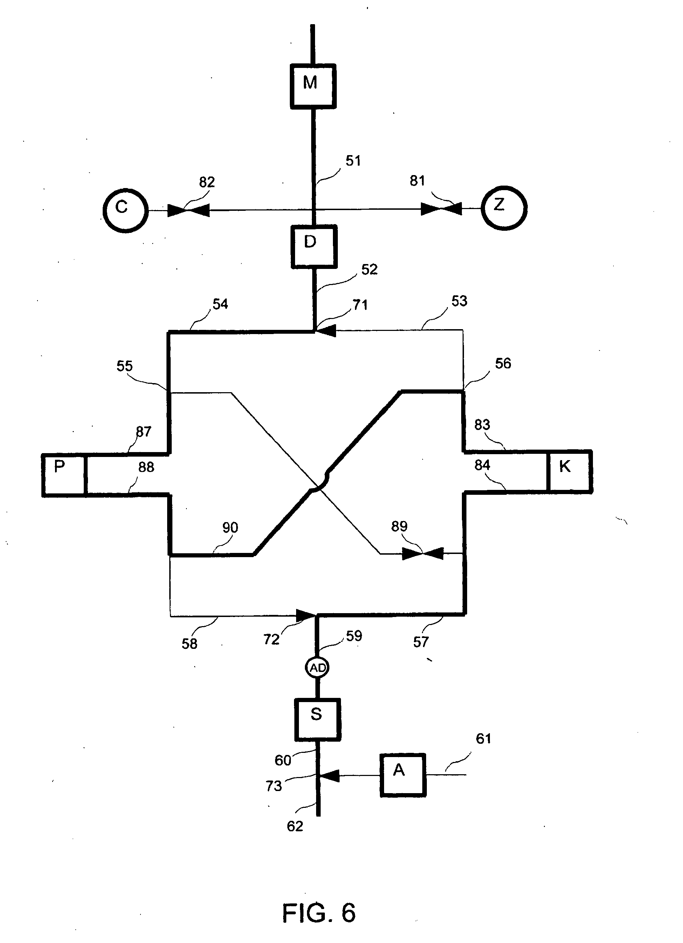

**FIG. 6** zeigt die Luftführung von **M** durch den Permeator **P** und anschließend den Katalysator **K**. Die am Sensor ankommende Luft wird dementsprechend als **MPK** bezeichnet. Öffnet man statt des Ventiles 51 entweder das Ventil 82 oder das Ventil 81, erhält man bei ansonsten gleicher Luftführung am Sensor Luft, die als **CPK** oder **ZPK** bezeichnet wird.

Die mit dem erfindungsgemäßen Gerät durchführbaren Messungen beruhen darauf, dass in bestimmten Abständen zwischen jeweils mindestens zwei der voran stehenden Schaltstellungen hin und her geschaltet wird. Bis sich am Sensor der zu dem jeweiligen Luftstrom gehörende Messwert einstellt, dauert es etwa 10 sec bis 120 sec. Dies hängt damit zusammen, dass der Sensor gegen Staub durch eine Membran geschützt ist und die Diffusion bis zum Konzentrationsausgleich abgewartet werden muss.

Es ist möglich, dass der Katalysator insbesondere in einer Anfangsphase von 1-3 Tagen permanent, jedoch abnehmend etwas Feuchtigkeit abgibt und damit den Messwert verfälscht. Schaltet man zwischen **Z** (also Nullluft) und **ZK** (also im Katalysator behandelte Nullluft) in einem Abstand von beispielsweise 90 sec hin und her, erhält man eine Spannungsdifferenz, bei der durch die Feuchtigkeitsabgabe des Katalysators der Wert **ZK** höher sein kann als **Z**, also sozusagen einen negativen Messwert. Die höhere Luftfeuchtigkeit in **ZK** kann also einen geringfügig höheren Messwert verursachen. Dieser "negative" Wert der Messwertedifferenz kann man als Feuchte-Kalibrierwert, also zur Kompensation des Messfehlers durch die Feuchtigkeitsabgabe des Katalysatormateriales am Messgerät einstellen.

Schaltet man zwischen der Stellung **C** und **CK** in einem Abstand von beispielsweise 90 sec hin und her, erhält man eine Spannungsdifferenz zur Kalibrierung, wobei **C** einen höheren und **CK** einen niedrigeren Spannungswert ergibt. Wenn zuvor am Gerät die Konzentration, die vom Sensorhersteller angegebene Sensorempfindlichkeit und der chemische Typ des Prüfgases eingestellt wurde, übernimmt das Gerät den gefundenen Wert als Kalibrierfaktor.

Schaltet man zwischen **C** und **Z** in einem Abstand von beispielsweise 90 sec hin und her, erhält man eine Spannungsdifferenz, die dem Wechsel zwischen **C** und **CK** entsprechen sollte, jedoch nur dann, wenn der Katalysator **K** voll funktionsfähig ist. Mit diesem Vergleich kann man daher also die Funktionsfähigkeit des Katalysators überprüfen.

Schaltet man zwischen der Stellung **C** und **Z** (oder **C** und **CK**) so hin und her, dass beispielsweise in jeweils einem Zyklus zunächst **CK** über 45 sec geöffnet bleibt und anschließend während 45 sec im Sekundentakt zwischen **C** und **CK** hin und hergeschaltet wird, erhält man nur den halben Konzentrationswert für **C.** Bei einer anderen Einstellung, nämlich beispielsweise 45 sec **CK**, dann 45 sec lang abwechselnd jeweils 1 sec **C** und 2 sec **CK**, erhält man 1/3 des Konzentrationswertes von **C** als Messergebnis, u.s.w. Unter der Abkürzung **"1C-2CK gegen CK"** wird dieses Beispiel nachfolgend bezeichnet.

Mit diesem Teil der Erfindung ist es also möglich, eine Funktion zu erstellen für die Abhängigkeit des Messwertes von der Konzentration zur Überprüfung der Linearität. Dazu kann man eine Reihe fahren von **"0C-10CK gegen CK"; "1C-9CK gegen CK";"2C-8CK gegen CK"** bis **....."9C-1CK gegen CK"; "10C-0CK gegen CK".** Anhand dieser Messwerte kann eine Linearisierungsfunktion ermittelt und gespeichert werden. Das Gerät kann die Richtigkeit dieser Linearisierungsfunktion nach dem eben Gesagten beliebig überprüfen.

Anhand dieser Linearisierungsfunktion kann das Gerät die gemessenen Werte des Messgases M gegen **MK** korrigieren. Das Gerät kann auch überprüfen, ob die für **C** gefundene Linearisierungsfunktion auch für **M** zutrifft, indem beispielsweise eine Verdünnungsreihe von **M-MK** gegen **MK** nach dem gleichen Schema wie im voran stehenden Abschnitt beschrieben, von **C-CK** gegen **CK** gefahren wird.

Die voran stehenden Messungen des Typs **C** und **Z** und deren katalytisch behandelte Nachfolgegase **CK** und **ZK** bedingen die Verwendung von Gasflaschen, da Prüfgase und Nullluft üblicherweise in Druckgasflaschen angeboten werden.

Dem Endanwender von Messgeräten ist es aber meist zu umständlich und teuer, mit Druckgasflaschen zu hantieren.

Es ist ein Teil dieser Erfindung, dass statt der Druckgasflaschen Permeatoren zum Kalibrieren verwendet werden und das Kalibriergas mittels Permeator aus der Messluft **M** erzeugt wird. Permeationsröhrchen können fertig kalibriert, beispielsweise von der Fa. Kin-Tec, USA, bezogen werden. Mittels Prüfgas **C** kann das Permeationsröhrchen auch nachkalibriert werden.

Mittels des Permeators **P** kann auch eine Verdünnungsreihe generiert werden. Es wäre aber falsch, wenn man pro Zyklus beispielsweise 45 sec lang in der Schaltstellung **MK** messen und dann 45 sec lang abwechselnd jeweils z.B. 0,3 sec **MKP** und z.B. 0,7 sec **MK** für beispielsweise 30 % des Abgabewertes aus dem Permeationsröhrchen messen wollte, da Permeationsröhrchen auch dann abgeben, wenn sie kurzfristig nicht durchströmt werden. Zeitweilige Unterbrechungen der Luftführung durch den Permeator **P** führen dann nur dazu, dass sich im Permeatorraum höhere Konzentrationen einstellen und beim Wiederdurchströmen demnach entsprechend höhere Konzentrationen abgegeben werden.

Die Verdünnungsreihe mittels Permeator wird daher so generiert, dass man pro Zyklus beispielsweise 45 sec lang in der Schaltstellung **MPK (****FIG. 6****)** misst, dann 45 sec lang abwechselnd jeweils z.B. 0,9 sec **MPK (****FIG. 6****)** und 2,1 sec **MKP (****FIG. 5****)**, um beispielsweise 70 % des Abgabewertes des Permeators zu erzeugen. Auf diese Weise werden in den Schaltpausen die nicht gewollten Anteile aus dem Permeator oxidiert und nicht akkumuliert, wie es der Fall wäre bei der Vorgehensweise, die im vorangegangenen Absatz erwähnt ist.

Eine Messung des vollen Wertes verläuft entsprechend vereinfacht durch Umschalten von **MPK (****FIG. 6****)** und **MKP (****FIG. 5****)** in beispielsweise 45 sec-Abständen.

Wegen der langen Lebensdauer von Permeationsröhrchen von bis zu mehreren Jahren liegt auf diese Weise eine neue Methode vor, das Messgerät regelmäßig, einfach und kostengünstig automatisch zu kalibrieren, bzw. linearisierend zu kalibrieren.

Das Permeationsröhrchen kann auch gelegentlich nachkalibriert werden durch eine Vergleichsmessung mit einer dazu anzuschließenden Prüfgasflasche, wobei das Prüfgas oder die Luft aus dem Permeator entsprechend dem voran erläuterten Verfahren so verdünnt werden können, dass sie im gleichen Konzentrationsbereich liegen und daher kein Linearisierungsfehler auftritt.

Schaltet man zur Messung der Messluft zwischen der Stellung **M** und **MK** in einem Abstand von beispielsweise 90 sec hin und her, erhält man eine Spannungsdifferenz, aus dem das Messgerät den Gehalt an dem jeweilig korrekt eingestellten Kohlenwasserstoff berechnet, wobei die Berechnung auf den Daten Feuchtigkeitskorrekturwert, Kalibrierfaktor, Responsefaktor und Kohlenwasserstofftyp beruht.

Das Gerät bietet die Möglichkeit zu überprüfen, ob der gefundene Messwert sich bereits in einem Messbereich befindet, der nichtlinear ist, bzw. ob der automatisch linearisierte Wert richtig ist. Dazu wird automatisch in Abständen eine Verdünnungsreihe generiert. Die Verdünnungsreihe wird so generiert, dass man pro Zyklus beispielsweise 45 sec lang in der Schaltstellung **MK (****FIG. 3****)** misst, dann 45 sec lang abwechselnd jeweils z.B. 0,9 sec **MK (****FIG. 3****)** und 2,1 sec **M (****FIG. 2****)** für beispielsweise 70 % des Vollwertes.

Im Messbereich unter 2 ppb ist es schwierig, eine korrekte Linearisierung anzuwenden, da die Abweichung von der Linearität mit abnehmender Signalstärke logarithmisch anwächst und dadurch kleinste Messungenauigkeiten entsprechend verstärkt werden. Daher ist vorgesehen, dass das Signal für die eigentliche Messung und für die Referenzmessung gleichermaßen um einen identischen Betrag angehoben wird. Damit schiebt man die Messung weiter in den Bereich höherer Signalstärke, wodurch man ein genaueres Messergebnis erhält.

Statt also die geringe Differenz zwischen einem sehr niedrigen **M** (Messluft) und **MK** (als Nullreferenz) zu messen, misst man stattdessen die Differenz zwischen **MP (****FIG. 4****)** und **MKP (****FIG. 5****)** als Nullreferenz. Es wird also sowohl auf die Messluft als auch auf die katalytisch behandelte Messluft jeweils die gleiche vom Permeator abgegebene Kohlenwasserstoffmenge dazugegeben. Die vom Permeator **P** abgegebene Kohlenwasserstoffmenge ist aufgrund der Auswahl des Permeationsröhrchens dabei gewollt wesentlich höher als die Konzentration von Kohlenwasserstoffen in **M.** Durch eine Verdünnungsreihe von **MP-MKP gegen MKP,** also z.B. im Wechsel 45 sec abwechselnd 1 sec **MP** und 4 sec **MKP** gegen 45 sec **MKP** (soll: 0,2 M) lässt sich die Verbesserung der Linearisierung überprüfen.

Trotz der Trägheit des Luftaustausches durch die den Sensor schützende Membran kommt es bei den schnellen Schaltwechseln im Sekundentakt während einer Messphase zu einem leicht sinusförmigem Signalverlauf, der durch mathematische Behandlung (Mittelwertbildung) geglättet wird.

Die Schaltdauer der jeweiligen Schaltzyklen kann von Hand am Gerät eingestellt werden. Das Gerät bietet aber auch die Möglichkeit, den optimalen Schaltzeitpunkt aus den generierten Messpunkten automatisch zu errechnen und entsprechend umzuschalten.

Zur katalytisch oxidativen Behandlung der kohlenwasserstoffhaltigen Luft ist die Beheizung auf mindestens 160°C nötig. Insbesondere für eine tragbare Ausführung des erfindungsgemäßen Gerätes bedeutet diese Aufheizung eine nur kurze Betriebsmöglichkeit mit (aufladbaren) Batterien. Erschwerend kommt die länge Aufheizzeit dazu. Eine vereinfachte Ausführung des Gerätes sieht daher statt des Katalysators einen Aktivkohleadsorber vor.

Da Aktivkohle neben Kohlenwasserstoffen auch Wasser adsorbiert, bewirkt der alternative Einsatz von Aktivkohle eine Herabsetzung der Genauigkeit der entsprechenden Messungen. Allerdings nimmt die Adsorptionsfähigkeit von Aktivkohle im Laufe der Zeit unkontrolliert ab.

Es wurde weiter oben bereits beschrieben, dass, wenn beide Seiten einer referenzierenden Messung um den gleichen Betrag an Kohlenwasserstoffen aus dem Permeator angereichert werden, sich die Messgenauigkeit steigern lässt, weil die Messung in den linearen Bereich verschoben wird. Zusätzlich wird dadurch aber auch der Einfluss der Luftfeuchtigkeit auf das Messergebnis herabgesetzt. Auf diese Weise können mittels Permeation auch die mit Aktivkohleadsorption anstatt Katalyse durchgeführten Messungen deutlich verbessert werden.

Man kann also mit der Schaltung **MP** (FIG. 4) gegen **MK'P** (FIG. 6; jedoch Aktivkohle **K'** statt Katalysator **K)** zu guten Messergebnissen kommen. Auch die vielfältigen oben beschriebenen und in **FIG. 2** **-** **FIG 6**. gezeigten Kombinationsmöglichkeiten sind gleichermaßen für **K'**, also den Aktivkohleadsorber, so wie oben für den Katalysator **K** beschrieben, möglich.

Es gibt auch eine vereinfachte Geräteausführung ohne Permeator, die entweder nur mit einem Katalysator und/oder nur mit einem Aktivkohleadsorber arbeitet.

Die oben beschriebenen Linearisierungsfunktionen durch Verdünnungsreihen lassen sich aber auch mit diesen vereinfachten Geräten durchführen.

Das erfindungsgemäße Messgerät beinhaltet die Möglichkeit, dass in der Software des Messgerätes als Bibliothek die Parameter der Inhaltsstoffe von zu messenden, handelsüblichen Ölen eingetragen und als Berechnungsgrundlage einbezogen werden.

Dazu werden von den handelsüblichen Kompressorenölen GC/MS-Analysen erstellt, und unter Berücksichtigung des jeweiligen Anteiles der Einzelkomponenten im Öl, deren jeweiligem Molekulargewicht und deren jeweiligem Responsefaktor als Konstanten im Programm eingestellt.

Das Messergebnis kann zur besseren Akzeptanz im Zweifelsfall mit einer etablierten Methode bestätigt werden. Dazu wird parallel ein Messwert nach DIN ISO 8573-5 mittels Kohlenwasserstoffsammelröhrchen und Analyse durch ein zertifiziertes Labor ermittelt und falls vom Anwender gewünscht, als "DIN ISO - Kalibrierwert" in einem Eingabefeld eingetragen.

Mit der Messmethode des vorliegenden Messgerätes gelingt es, durch alternierende Ventilschaltungen Signaldifferenzen und dadurch Messergebnisse zu erzeugen und dabei gleichzeitig
a) die Sensordrift auszugleichen,
b) den wechselnden Feuchtigkeitsgehalt der Messluft auszugleichen,
c) die Funktionsfähigkeit des Katalysators zu überwachen,
d) die Messgenauigkeit durch Zumischen zu erhöhen,
e) die Funktionsfähigkeit des Permeationsröhrchen zu überwachen und
f) den dauernden Abgleich mit der Messmethode nach DIN ISO 8573-5 zu ermöglichen.

In einer erweiterten Ausführung des Messgerätes bildet das Messgerät eine Baueinheit mit einem katalytisch oxidativen Behandlungsgerät zur Entfernung von Kohlenwasserstoffen oder reduzierenden Gasen aus großen Mengen genutzter Luft oder Druckluft. Dabei fließen also beispielsweise 1 m³ / min durch den Katalysator des Behandlungsgerätes für genutzte Druckluft und 2 1 / min durch den eigenen kleinen Katalysator für das Messgerät. Der Vorteil ist dabei, dass der kleine Katalysator des Messgerätes in den großen Katalysator mit eingebaut ist und mit geheizt wird. In der praktischen Ausführung befindet sich dann im beheizten Katalysatorbett lediglich eine separierendes Rohr mit einem eigenen Ausgang.

Das Behandlungsgerät enthält einen Wärmetauscher zur Rückgewinnung der eingesetzten Heizenergie und zur Aufheizung der zugeführten kohlenwasserstoffhaltigen Luft. Während bei größeren Ausführungen bevorzugt Plattenwärmetauscher verwendet werden, werden in kleinen Ausführungen des Behandlungsgerätes bevorzugt Rohr-in-Rohr-Wärmetauscher eingesetzt.

Die Dimensionierung der Katalysatormengen und die durchströmenden Luftmengen durch Behandlungsanlage und Messgerät sind so gewählt, dass der Katalysator des Messgerätes erheblich weniger belastet wird. Der Katalysator des Messgerätes sollte im Mengenverhältnis Kohlenwasserstoff zu Katalysator im Vergleich bevorzugt nur mit bis zu einem Zehntel im Vergleich zum Katalysator der Behandlungsanlage beaufschlagt werden.

Sobald im Laufe der Zeit die oxidative Leistung des Katalysators der Behandlungsanlage abnimmt, differieren zunehmend die ursprünglich gleichen Kohlenwasserstoffwerte des Messluftstroms und des Referenzluftstroms, deren Differenz vom Messgerät ermittelt wird. Dadurch wird die abnehmende Leistung des Katalysators der Behandlungsanlage vom Messgerät festgestellt und überwacht.

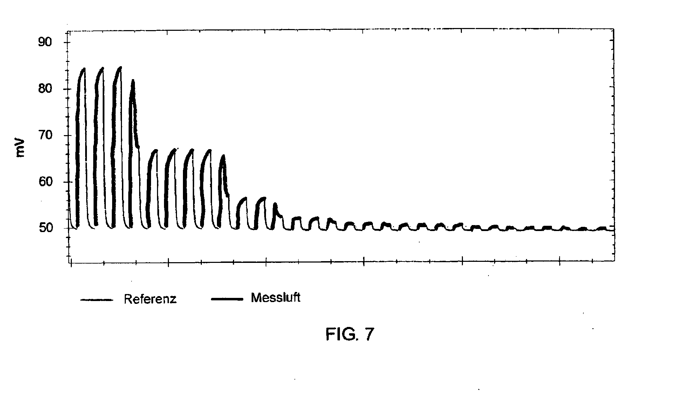

In FIG. 7 ist der Messkurvenverlauf bei einer beispielhaften Messung mit Verdünnungsreihen für **C-CK gegen CK** gezeigt. Nach dem Umschalten des Magnetventiles auf **C-CK** erfolgt ein steiler Anstieg, bis sich der jeweilige Verdünnungswert für **C-CK** eingestellt hat; nach Umschalten auf **CK** fällt der Messwert wieder ab. Nach jeweils der 3., 8., 11., 14., 17., 22., 24., und der 28 Messung wurde jeweils stärker verdünnt, d.h. das Verhältnis der Einschaltdauer von **C** wird immer kürzer. Die jeweils erste Messung nach dem jeweiligen Umschalten zur nächsten Verdünnung soll nicht beachtet werden.

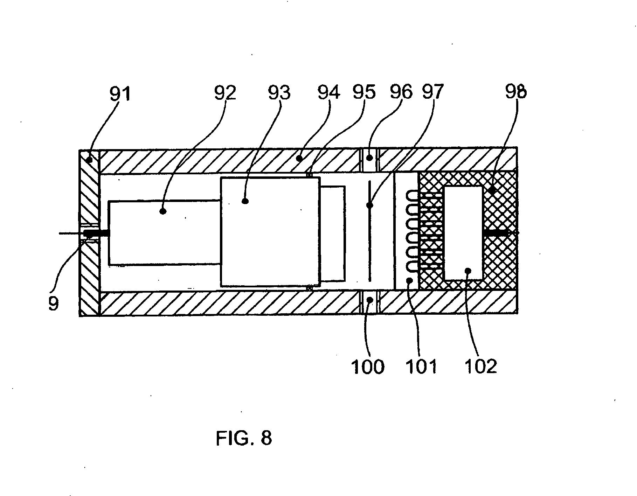

Ebenfalls Gegenstand der vorliegenden Erfindung ist eine Ausführung, bei der der Photokatalysator eine Baueinheit mit dem Photoionisationsdetektor als Messkammer bildet (siehe **FIG. 8**). Diese Messkammer **91** ist vorzugsweise so ausgeführt, dass durch den Einbau eines Photoionisationsdetektors **93** mit einem AD Wandler **92** und einer UV - Photoeinheit **98** ein Hohlraum von < 1 cm³ Rauminhalt gebildet wird, der eine photokatalytisch aktive Folie **97** enthält, Die Folie 97 wird durch eine Kammerwand **101** aus transparentem Kunstoff von mehreren UV-LEDs angestrahlt, während die Frontscheibe des Photoionisationsdetektors **93** eine andere Kammerwand bildet. Die Gasfüllung dieser Kammer wird periodisch über die Öffnungen **96** und **100**, die mit Fließwiderständen bestückt sind, ausgetauscht.

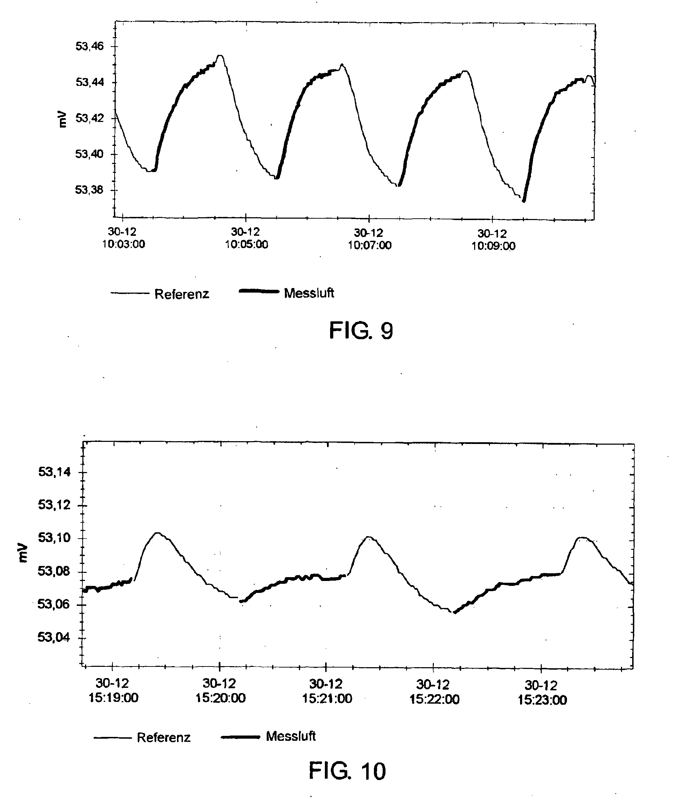

Eine typische Messung von Druckluft mit einer solchen Ausführung des Messgerätes zeigt **FIG. 9****.** Die Druckluft aus einem ölbetriebenen Kompressor (7 bar) wurde mittels Kältetrockner, Vorfilter, Wasserabscheider und Aktivkohle vorbehandelt. Der Restöldampfgehalt dieser Druckluft liegt in der Regel deutlich unter 0,01 mg/m³ Normalluft. Auf den Anstieg des Messignales während der Messluftphase **M** folgt ein Absinken des Signales während der Katalysatorluftphase **MK**. Zu Beginn der Katalysatorluftphase ist deutlich ein "Buckel" zu erkennen. Dass dieser Buckel von abgegebenem Wasser verursacht wird, zeigt folgendes Beispiel. Misst man mit der gleichen Anordnung statt der beschriebenen Druckluft geringfügig angefeuchtete Nullluft, wird, wie **FIG. 10** zeigt, der Buckel sehr groß und es folgt gegen Ende der Katalysatorluftphase ein entsprechend tiefes Tal. Während der Messluftphase steigt der Wert wieder an. Obwohl diese Nullluft keine Kohlenwasserstoffe enthält, entsteht also durch das Verhalten des Hopcalit-Katalysators ein künstlicher positiver Messwert. Zu Beginn der Katalysatorluftphase entsteht durch abgegebenes Wasser ein Buckel, gegen Ende wird Wasser adsorbiert wodurch der Wert entsprechend kleiner wird.

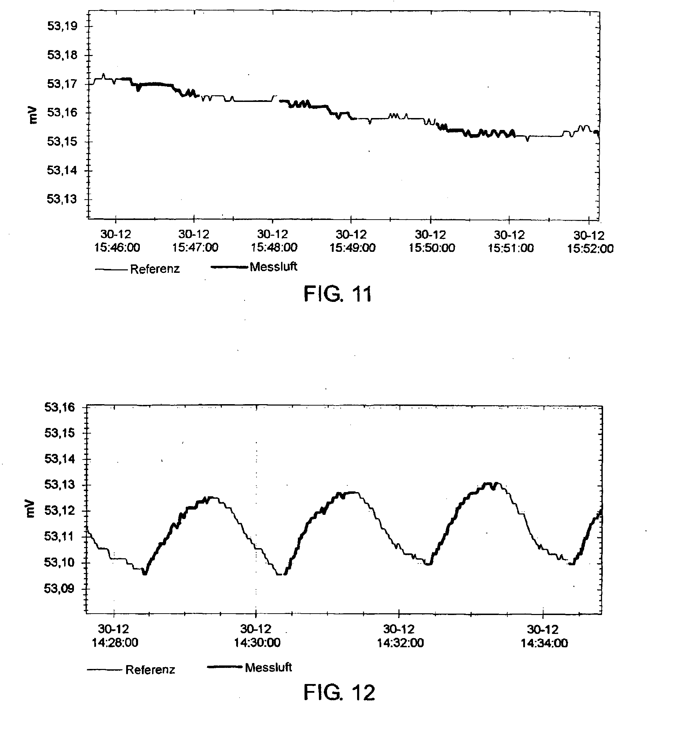

Durch die Verwendung des Kompensators **AD** wird dieser Effekt beseitigt. In **FIG. 11** ist die Auswirkung des Kompensators auf die Messung entsprechend dem Messdiagramm von **FIG. 10** mit angefeuchteter Nullluft zu erkennen. Der jetzt resultierende Messwert liegt unter der Grenzempfindlichkeit des Sensors, sodass nur noch ein Rauschen von etwa 2 Microvolt zu erkennen ist, bedingt durch die Auflösung des AD-Wandlers. Lediglich die Drift des Sensors verändert das Messsignal. Der angezeigte, driftkompensierte Messwert liegt unter 0,001 mg/m³.

In **FIG. 12** ist die Auswirkung des Kompensators auf das Messignal bei der Messung von Druckluft analog **FIG. 9** zu erkennen. Hier wurde eine Menge von 2 g poröser Aluminiumoxidperlen zur Kompensation gegenüber einer Menge von 5 g Hopcalitgranulat in einem von außen auf 200 ° C beheizten 8 mm Edelstahlrohr von 30 cm Länge verwendet. Der Buckel ist bei dieser Menge von 2 g bereits vollständig verschwunden.

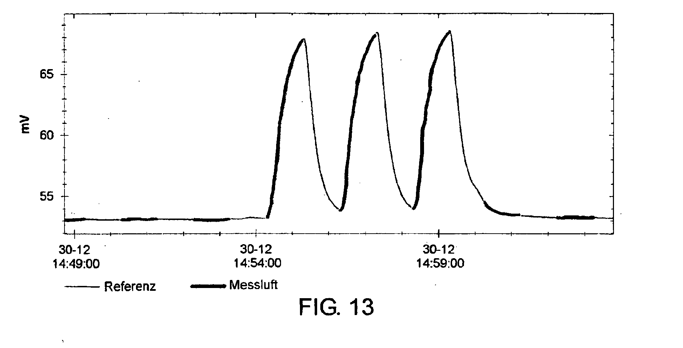

Hier stellte sich die Frage, inwieweit der Kompensator unerwünschter Weise auch den Restölgehalt kompensiert. Überraschenderweise wurde gefunden, dass sich nur ein sehr kleiner Effekt der ungewünschten "Kompensation" des Öldampfes einstellt, d.h. der Öldampf kann das Kompensationsmaterial ohne nennenswerte Speicherung passieren. Offenbar funktionieren derartige poröse Materialien nur bei deutlich höheren Konzentrationen als brauchbare Adsorber. Zur Bestätigung wurden Messungen entsprechend **FIG. 13** durchgeführt. Während einer Messung von Druckluft (< 0,01 mg/ m³) mit entsprechend wenigen ppb Kohlenwasserstoffen wurde das Messgerät für 3 Messzyklen mit einem deutlich stärker konzentrierten Prüfgas mit einer Konzentration von 0,5 ppm an Kohlenwasserstoffen beaufschlagt. In **FIG. 13** sieht man die vergleichsweise schwachen Signale der Druckluft, dann drei starke Signale für das Prüfgas und anschließend sofort wieder die kleinen Signale für die Druckluft und bei den letzteren - als Kernaussage - keinen erhöhten Wert für den Gehalt im Anschluss nach den starken Messsignalen. Da der Maßstab bei dieser Abbildung sehr viel kleiner ist, kann man bei dieser Abbildung der **FIG. 13** den Wechsel der Signalstärke bei der Messung der Druckluft in der Abbildung optisch nicht mehr erkennen. Die Signale links und rechts der drei starken Signale in **FIG. 13** entsprechen aber denen in **FIG. 12**.

## Patentansprüche

1. Messgerät zum Erfassen des Gehaltes von Öl, Kohlenwasserstoffen und oxidierbaren Gasen in Luft oder Druckluft, **dadurch gekennzeichnet, dass** das Messgerät einen Luft- bzw. Druckluftanschluß (M) und einen sich daran anschließenden Durchftussbegrenzer (D) sowie einen sich daran anschließenden beheizbaren Oxidationskatalysator (K) aufweist, an den sich ein Photoionisationsdetektor (S) anschließt, wobei schaltbare Mittel (90, 72) zum Leiten der Luft bzw. Druckluft über den Oxidationskatalysator (K) und an diesem vorbei direkt zum Photoionisationsdetektor (S) vorgesehen sind und dass zwischen dem Luft- bzw. Druckluftanschluß (M) und dem Durchflussbegrenzer (D) ein schaltbarer Prüfgaseinlaß (C) angeordnet ist.

2. Messgerät zum Erfassen des Gehaltes von Öl, Kohlenwasserstoffen und oxidierbaren Gasen in Luft oder Druckluft, **dadurch gekennzeichnet, dass** das Messgerät einen Luft- bzw. Druckluftanschluß (M) und einen sich daran anschließenden Durchflussbegrenzer (D) sowie einen sich daran anschließenden UV-Photokatalysator (K) aufweist, an den sich ein Photoionisationsdetektor (S) anschließt, wobei schaltbare Mittel (90, 72) zum Leiten der Luft bzw. Druckluft über den UV-Photokatalysator (K) und an diesem vorbei direkt zum Photoionisationsdetektor (S) vorgesehen sind und dass zwischen dem Luft- bzw. Druckluftanschluß (M) und dem Durchflussbegrenzer (D) ein schaltbarer Prüfgaseinlaß (C) angeordnet ist.

3. Messgerät zum Erfassen des Gehaltes von Öl, Kohlenwasserstoffen und oxidierbaren Gasen in Luft oder Druckluft, **dadurch gekennzeichnet, dass** das Messgerät einen Luft- bzw. Druckluftanschluß (M) und einen sich daran anschließenden Durchflussbegrenzer (D) und einen sich daran anschließenden Aktivkohleadsorber aufweist, an den sich ein Photoionisationsdetektor (S) anschließt, wobei schaltbare Mittel (90, 72) zum Leiten der Luft bzw. Druckluft über den Aktivkohleadsorber und an diesem vorbei direkt zum Photoionisationsdetektor (S) vorgesehen sind und dass zwischen dem Luft- bzw. Druckluftanschluß (M) und dem Durchflussbegrenzer (D) ein schaltbarer Prüfgaseinlaß (C) angeordnet ist.

4. Messgerät gemäß Anspruch 2, **dadurch gekennzeichnet, daß** eine Messkammer vorgesehen ist, die einen in die Kammermitte ragenden Detektoreingang für einen Photoionisationsdetektor aufweist, bei der eine Kammmerwand aus mit UV-LEDs bestücktem transparentem Kunststoff besteht, der von Kammer, Detektor und der transparenten Kunststoffwand gebildete Hohlraum kleiner als 1 ml ist und eine Öffnung mit Fließwiderstand für den periodischen Gasaustausch in dem Hohlraum vorgesehen ist.

5. Messgerät gemäß einem der Ansprüche 1, 2 oder 3, **dadurch gekennzeichnet, dass** parallel zu dem Oxidationskatalysator (K) bzw. dem Aktivkohleadsorber mindestens ein Permeator (P) zum Freisetzen einer über die Zeit konstanten, definierten Kohlewasserstoffmenge angeordnet ist, wobei schaltbare Mittel (89, 72) zum Leiten der Luft bzw. Druckluft über den Permeator (P) und an diesem vorbei direkt zum Photoionisationsdetektor (S) vorgesehen sind.

6. Messgerät gemäß einem der Ansprüche 1 bis 5, **dadurch gekennzeichnet, dass** zwischen dem Luft- bzw. Druckluftanschluß (M) und dem Durchflussbegrenzer (D) ein schaltbarer Nulllufteinlaß (Z) angeordnet ist.

7. Messgerät gemäß einem der Ansprüche 1 bis 6, **dadurch gekennzeichnet, dass** im Anschluss an den Photoionisationsdetektor (S) ein schaltbarer Anschluß (73) zu einem Aktivkohleröhren (A) vorgesehen ist.

8. Messgerät gemäß einem der Ansprüche 1 bis 7, **dadurch gekennzeichnet, dass** dem Photoionisationsdetektor (S) ein Kompensator (AD) für die Luftfeuchtigkeit vorgeschaltet ist.

9. Verfahren zum Erfassen des Gehaltes von Öl, Kohlenwasserstoffen und oxidierbaren Gasen in Luft oder Druckluft unter Verwendung eines Messgerätes gemäß den Ansprüchen 1 bis 8, **dadurch gekennzeichnet, dass** zur Durchführung einer referenzierenden Messung während der Messzeit von kohlenwasserstoffhaltigen Luft- oder Druckluftströmen ein variabler Anteil der Kohlenwasserstoffe katalytisch oxidiert wird und auf diese Weise Verdünnungsreihen erzeugt werden.

10. Verfahren zum Erfassen des Gehaltes von Öl, Kohlenwasserstoffen und oxidierbaren Gasen in Luft oder Druckluft gemäß Anspruch 9, **dadurch gekennzeichnet, dass** zur Durchführung einer referenzierenden Messung während der Messzeit der kohlenwasserstoffhaltigen Luft ein variabler Anteil Nullluft zur kohlenwasserstoffhaltigen Luft zugemischt wird und auf diese Weise Verdünnungsreihen erzeugt werden.

11. Verfahren gemäß einem der Ansprüche 9 oder 10, **dadurch gekennzeichnet, dass** zum Erhöhen der Meßgenauigkeit durch den Permeator die zu messende kohlenwasserstoffhaltige Luft, als auch die katalytisch behandelte Luft, bzw. Nullluft, in gleicher Menge mit Kohlenwasserstoff angereichert wird, um den Messbereich in den linearen Messbereich zu verschieben.

## Claims

1. Measuring instrument for detecting the content of oil, hydrocarbons and oxidizable gases in air or compressed air, **characterized in that** the measuring instrument has an air or compressed-air connection (M) and, connected thereto, a flow limiter (D) to which a thermal oxidation catalyst (K) is connected that is connected in turn with a photoionisation detector (S), switchable means (90, 72) being provided for routing the air or compressed air over the oxidation catalyst (K) or past it directly to the photoionisation detector (S), and that a switchable test-gas inlet (C) is fitted between the air or compressed-air connection (M) and the flow limiter (D).

2. Measuring instrument for detecting the content of oil, hydrocarbons and oxidizable gases in air or compressed air, **characterised in that** the measuring instrument has an air or compressed-air connection (M) and, connected thereto, a flow limiter (D) to which a UV photocatalyst (K) is connected that is connected in turn with a photoionisation detector (S), switchable means (90, 72) being provided for routing the air or compressed air over the UV photocatalyst (K) or past it directly to the photoionisation detector (S), and that a switchable test-gas inlet (C) is fitted between the air or compressed-air connection (M) and the flow limiter (D).

3. Measuring instrument for detecting the content of oil, hydrocarbons and oxidizable gases in air or compressed air, **characterised in that** the measuring instrument has an air or compressed-air connection (M) and, connected thereto, a flow limiter (D) to which an activated charcoal adsorber is connected that is connected in turn with a photoionisation detector (S), switchable means (90, 72) being provided for routing the air or compressed air over the activated charcoal adsorber or past it directly to the photoionisation detector (S), and that a switchable test-gas inlet (C) is fitted between the air or compressed-air connection (M) and the flow limiter (D).

4. Measuring instrument according to claim 2, **characterised in that** a measuring chamber is provided which has, projecting into the centre thereof, a detector input for a photoionisation detector, and whose one chamber wall consists of transparent plastic fitted with UV LEDs, that the hollow space formed by the chamber, detector and the transparent plastic wall is smaller than 1 ml and that an opening with a flow resistor is provided for periodically exchanging the gas in the hollow space.

5. Measuring instrument according to any one of the claims 1, 2 or 3, **characterised in that**, in addition to the oxidation catalyst (K) or the activated charcoal adsorber, at least one permeator (P) for releasing a temporally constant, defined amount of hydrocarbon is installed, with switchable means (89, 72) being provided for routing the air or compressed air via the permeator (P) or past it directly to the photoionisation detector (S).

6. Measuring instrument according to any one of the claims 1 to 5, **characterised in that** a switchable zero-air inlet (Z) is fitted between the air or compressed-air connection (M) and the flow limiter (D).

7. Measuring instrument according to one of the claims 1 to 6, **characterised in that** a switchable connection (73) to an activated-charcoal tube (A) is provided subsequent to the photoionisation detector (S).

8. Measuring instrument according to any one of the claims 1 to 7, **characterised in that** an air-moisture compensator (AD) is fitted upstream of the photoionisation detector (S).

9. Method for detecting the content of oil, hydrocarbons and oxidizable gases in air or compressed air using a measuring instrument according to the claims I to 8, **characterised in that**, for the purpose of carrying out a referencing measurement while hydrocarbon-containing-air or compressed-air streams are being analysed, a variable proportion of the hydrocarbons is catalytically oxidized and, in this way, dilution series are generated.

10. Method for detecting the content of oil, hydrocarbons and oxidizable gases in air or compressed air according to claim 9, **characterised in that**, for the purpose of carrying out a referencing measurement while hydrocarbon-containing air is being analysed, a variable proportion of zero air is mixed with the hydrocarbon-containing air and, in this way, dilution series are generated.

11. Method according to either one of the claims 9 or 10, **characterised in that**, in order to increase the measuring accuracy, both the hydrocarbon-containing air to be analysed and the catalytically treated air or zero air are enriched by way of the permeator with equal amounts of hydrocarbon so as to shift the measuring range into the linear measuring range.

## Revendications

1. Appareil de mesure pour détecter la teneur en huiles, hydrocarbures et gaz oxydables dans de l'air ou de l'air comprimé, **caractérisé en ce que** l'appareil de mesure comprend un raccord d'air ou d'air comprimé (M) et un limitateur de débit (D) raccordé à celui-ci ainsi qu'un catalyseur d'oxydation (K) pouvant être chauffé se raccordant à ce dernier et auquel se raccorde un détecteur de photoionisation (S), des moyens commutables (90, 72) étant prévus pour conduire l'air ou l'air comprimé sur le catalyseur d'oxydation (K) et, en le contournant, directement au détecteur de photoionisation (S), et **en ce qu'**une entrée de gaz de contrôle (C) commutable est disposée entre le raccord d'air ou d'air comprimé (M) et le limitateur de débit (D).

2. Appareil de mesure pour détecter la teneur en huiles, hydrocarbures et gaz oxydables dans de l'air ou de l'air comprimé, **caractérisé en ce que** l'appareil de mesure comprend un raccord d'air ou d'air comprimé (M) et un limitateur de débit (D) raccordé à celui-ci ainsi qu'un photocatalyseur à UV (K) se raccordant à ce dernier et auquel se raccorde un détecteur de photoionisation (S), des moyens commutables (90, 72) étant prévus pour conduire l'air ou l'air comprimé sur le photocatalyseur à UV (K) et, en le contournant, directement au détecteur de photoionisation (S), et **en ce qu'**une entrée de gaz de contrôle (C) commutable est disposée entre le raccord d'air ou d'air comprimé (M) et le limitateur de débit (D).

3. Appareil de mesure pour détecter la teneur en huiles, hydrocarbures et gaz oxydables dans de l'air ou de l'air comprimé, **caractérisé en ce que** l'appareil de mesure comprend un raccord d'air ou d'air comprimé (M) et un limitateur de débit (D) raccordé à celui-ci ainsi qu'un adsorbant à charbon actif se raccordant à ce dernier et auquel se raccorde un détecteur de photoionisation (S), des moyens commutables (90, 72) étant prévus pour conduire l'air ou l'air comprimé sur l'adsorbant à charbon actif et, en le contournant, directement au détecteur de photoionisation (S), et **en ce qu'**une entrée de gaz de contrôle (C) commutable est disposée entre le raccord d'air ou d'air comprimé (M) et le limitateur de débit (D).

4. Appareil de mesure selon la revendication 2, **caractérisé en ce qu'**il est prévu une chambre de mesure qui présente une entrée de détecteur pour un détecteur de photoionisation, laquelle entrée saille au centre de la chambre de mesure, dans laquelle chambre une paroi de la chambre est composée d'une matière plastique transparente munie de LED UV, la cavité formée par la chambre, le détecteur et la paroi en matière plastique transparente est inférieure à 1 ml et une ouverture avec une résistance à l'écoulement est prévue pour échanger périodiquement le gaz dans la cavité.

5. Appareil de mesure selon l'une des revendications 1, 2 ou 3, **caractérisé en ce qu'**au moins un perméateur (P) est disposé parallèlement au catalyseur d'oxydation (K) ou à l'adsorbant à charbon actif pour libérer une quantité définie d'hydrocarbure constante dans le temps, des moyens commutables (89, 72) étant prévus pour conduire l'air ou l'air comprimé sur le perméateur (P) et, en le contournant, directement au détecteur de photoionisation (S).

6. Appareil de mesure selon l'une des revendications 1 à 5, **caractérisé en ce qu'**une entrée permutable d'air de calibrage est prévue entre le raccord d'air ou d'air comprimé (M) et le limitateur de débit (D).

7. Appareil de mesure selon l'une des revendications 1 à 6, **caractérisé en ce qu'**un raccord (73) permutable menant à un tube à charbon actif (A) est prévu à la suite du détecteur de photoionisation (S).

8. Appareil de mesure selon l'une des revendications 1 à 7, **caractérisé en ce qu'**un compensateur (AD) de l'humidité de l'air est raccordé en amont du détecteur de photoionisation (S).

9. Procédé pour détecteur la teneur en huiles, hydrocarbures et gaz oxydables dans de l'air ou de l'air comprimé en utilisant un dispositif de mesure selon l'une des revendications 1 à 8, **caractérisé en ce que** pour réaliser une mesure de référence pendant la durée de la mesure de courants d'air ou d'air comprimé contenant des hydrocarbures, une fraction variable des hydrocarbures est oxydée de façon catalytique et des séries de dilutions sont ainsi réalisées,

10. Procédé pour déterminer la teneur en huiles, hydrocarbures et gaz oxydables dans de l'air ou de l'air comprimé selon la revendication 9, **caractérisé en ce que** pour réaliser une mesure de référence pendant la durée de la mesure de l'air contenant des hydrocarbures, une fraction variable d'air de calibrage est mélangée à l'air contenant des hydrocarbures et des séries de dilutions sont ainsi réalisées.

11. Procédé selon l'une des revendications 9 ou 10 **caractérisé en ce que** pour augmenter la précision l'air contenant des hydrocarbures mais également l'air traité de façon catalytique ou l'air de calibrage sont enrichis en même quantité en hydrocarbures par le perméateur en vue de déplacer la zone de mesure dans la zone de mesure linéaire.
